Europäisches Patentamt

European Patent Office

Office européen des brevets

(19)

(11) **EP 0 440 373 B1**

(12) **EUROPEAN PATENT SPECIFICATION**

(45) Date of publication and mention
of the grant of the patent:
**23.04.1997 Bulletin 1997/17**

(51) Int Cl.[6]: **C12N 5/08, A61K 35/14**

(21) Application number: **91300554.2**

(22) Date of filing: **24.01.1991**

(54) **Method of activating cytolytic activity of lymphocytes using anti-CD28 antibody**

Verfahren zur Aktivierung der zytolytischen Wirkung von Lymphozyten unter Verwendung von anti-CD28-Antikörper

Procédé d'activation de l'activité cytolytique des lymphocytes utilisant un anticorps contre CD28

(84) Designated Contracting States:
**AT BE CH DE DK ES FR GB GR IT LI LU NL SE**

(30) Priority: **25.01.1990 US 471934**

(43) Date of publication of application:
**07.08.1991 Bulletin 1991/32**

(73) Proprietor: **Bristol-Myers Squibb Company
New York, N.Y. 10154 (US)**

(72) Inventor: **Ledbetter, Jeffery A.
Seattle, Washington 98177 (US)**

(74) Representative: **Jones, Alan John et al
CARPMAELS & RANSFORD
43 Bloomsbury Square
London, WC1A 2RA (GB)**

(56) References cited:
**EP-A- 0 360 205          WO-A-90/05541**

- **THE JOURNAL OF IMMUNOLOGY, vol. 140, no. 6, 15th March 1988, The American Association of Immunologists; N. K. DAMLE et al, pages 1753-1761**
- **PROC. NATL. ACAD. SCI. USA, vol. 84, no. 13, July 1987, Washington, DC, US; G. JUNG et al, pages 4611-4615**

**Description**

FIELD OF THE INVENTION

This invention relates to methods for potentiating cytolytic activity of lymphocytes, and more specifically to the use of anti-CD28 antibody reactive with receptors on the surface of lymphocytes.

BACKGROUND OF THE INVENTION

One of the promising methods of therapy for cancer is immunotherapy, in which leukocytes, particularly lymphocytes, are stimulated to attack tumor cells. One such method is known as adoptive immunotherapy, in which lymphocytes are stimulated in vitro by interleukin-2 (IL-2) to grow and become cytolytic and are then reintroduced into the organism to fight the tumor cells. (Rosenberg et al., Science 223:1318-1321 (1986)). International Patent Application WO90/05541 describes an in vivo method for stimulating T lymphocytes to produce higher levels of lymphokines.

Methods are known for stimulating lymphocytes to attack tumor cells. For example, CD3-positive T cells can be targeted to lyse tumor cells using heteroconjugates prepared by linking monoclonal antibodies (mAbs) to CD3 and a mAb reactive with a tumor-associated target cell antigen, after incubation with anti-CD3 mAb or with IL-2. (Jung et al., Proc. Nat. Acad. Sci. (U.S.A) 83: 4479 (1986); Jung et al., Proc. Nat. Acad. Sci. (U.S.A.) 84: 4611 (1987); Titus et al., J. Immunol. 138:4018 (1987); Perez et al., J. Immunol. 137:2069 (1986); Perez et al., Nature 316: 354 (1985)). Likewise, heteroconjugates consisting of anti-CD3 and anti-viral mAbs have been described that induce specific lysis of virus-infected cells (Zarling et al., J. Immunol. 140:2609 (1988); Staerz, et al. Eur. J. Immunol. 17:571 (1987)). These cytolytic cells are referred to as "CD3-dependent" because they require contact with the CD3 receptor to stimulate lytic activity. Specificity of CD3-dependent lysis can also be conferred by conjugating a hormone, such as melanocyte-stimulating hormone, to an anti-CD3 mAb (Liu et al., Science 239:395 (1988)).

Activated T-cells have been reported to be lytic to tumor cells without requiring stimulation of CD3 using Phytohemaglutinin (PHA) stimulation of NK-depleted T cells, or IL-2 stimulation of purified T cells. (Thiele, et al. J. Immunol. 140:3253 (1988); Calamonici et al., J. Immunol. 140:2527 (1988); Damle et al., J. Immunol. 137:2814 (1986); Moriyama et al., Cell. Immunol. 111:482 (1988)). A natural killer (NK)-like activity of $CD3^+$ T-cell clones and leukemic cells has also been observed. (Binz et al., J. Exp. Med. 157:1252 (1983); Hercend et al., Nature 301:158 (1983); Irle et al., Human Immunol. 11:183 (1984); Brooks et al., J. Immunol. 138:1331 (1987); Roberts et al., Eur. J. Immunol. 15:448 (1985)). The killing generated after activation is not restricted by the major histocompatibility complex (MHC) and is directed to multiple tumor targets (Thiele et al., supra; Calamonici et al., supra; Damle et al., supra). Such killing is not dependent on CD3 interaction with the tumor cells but may require surface expression of the CD3 T-cell receptor complex (CD3/Ti). (Thiele, et al. supra). This killing is referred to as "CD3-independent" lytic activity.

It would, therefore, be desirable to find a novel and efficient method of stimulating T-cells to develop CD3-independent cytolytic activity to attack tumor cells.

SUMMARY OF THE INVENTION

Accordingly, the present invention provides a method for potentiating the development of CD3 independent cytolytic activity of lymphocytes to produce cytolytic lymphocytes by contacting the lymphocytes with antibody reactive with CD28 receptor on the surface of the lymphocytes. The cytolytic activity generated by stimulation of the lymphocytes is CD3 heteroconjugate-independent because the activity does not require stimulation of the CD3/Ti receptor complex, and therefore does not require the use of heteroconjugates containing antibody reactive with the CD3-Ti receptor complex.

The antibody reactive with CD28 receptor is typically a monoclonal antibody. The monoclonal antibody can be an IgG2a antibody, such as monoclonal antibody 9.3.

The method further includes the step of contacting the lymphocytes with a second antibody reactive with CD2 receptor. The method can also further comprise the step of contacting the lymphocytes with interleukin-2 (IL-2).

For maximum cytolytic response, the CD28 receptor is aggregated on the surface of the lymphocytes. The CD28 receptor can be aggregated on the surface of the lymphocytes by crosslinking the antibody reactive with CD28 on the surface of the lymphocytes, for example using a second antibody reactive with the antibody reactive with the CD28 receptor. The second antibody can be a rat monoclonal antibody binding to mouse K light chains, such as monoclonal antibody 187.1. CD28 receptor aggregation can also be induced by a high molecular weight conjugate, for example containing anti-CD28 antibody bound to anti-CD28 antibody, such as 9.3 monoclonal antibody bound to 9.3 monoclonal antibody, (9.3 X 9.3) or by use of immobilized anti-CD28 antibody. The lymphocytes can additionally be contacted with IL-2.

Another important aspect of the present invention is a method for treating cancer in a subject. The method comprises the steps of:

(1) contacting lymphocytes in vitro with an antibody reactive with CD28 receptor to potentiate the development of CD3 heteroconjugate-independent cytolytic activity of the lymphocytes to produce cytolytic lymphocytes; and

(2) introducing the cytolytic lymphocytes into a subject to kill cancer cells in the subject.

BRIEF DESCRIPTION OF THE DRAWINGS

Figure 1 is graphs showing treatment of CD16 mAb plus C' to NK cells activated by six methods tested for their ability to lyse in the presence of anti-CD3 (G19-4)/L6 heteroconjugate ( □—□ ), unconjugated G19-4 and L6 ( ⊙—⊙ ), or alone ( ●—● ), as described in the Example, infra.

Figure 2 is graphs showing the results of treating T-cells with increasing concentrations of IL-2 in the presence of an anti-CD3 mAb (G19-4)/L6 heteroconjugate ( □—□ ), unconjugated mAb G19-4 and L6 ( ⊙—⊙ ) or alone ( ●—● ) as described in the Example, infra.

DETAILED DESCRIPTION OF THE INVENTION

In order that the invention herein described may be more fully understood, the following detailed description is set forth.

The present invention provides a method of stimulating lymphocytes to kill tumor cells using anti-CD28 mAb reactive with the lymphocyte receptor CD28. Although it is believed that agents such as IL-2, anti-CD2 mAb or immobilized anti-CD3 mAb must be present when the T cells are originally stimulated by the anti-CD28 mAb to induce the cytolytic activity, it has been found, unexpectedly, that lymphocytes can be efficiently stimulated to attack and lyse tumor cells by contact with anti-CD28 mAb in the absence of CD3 heteroconjugates during the killing or "effector" phase of the lysis. The activity is not inhibited by anti-CD3 mAb. This cell lysing activity stimulated by the contact of T-cell lymphocytes with anti-CD28 antibody is referred to herein as "cytolytic activity". The cytolytic activity stimulated by anti-CD28 mAb is defined herein as "CD3 heteroconjugate independent" during the cell killing phase because a heteroconjugate containing an antibody reactive with the CD3 receptor on T cells and an antibody reactive with tumor-associated antigen for targeting to the tumor cells is not required during this phase.

1. General Stimulation Methods

In general, the development of cytolytic activity can be stimulated by contacting the lymphocytes with anti-CD28 antibody, preferably in the presence of small amounts of IL-2, or in the presence of anti-CD2 antibody. Preferably, the lymphocytes are T-cell lymphocytes. The anti-CD28 antibody can be a polyclonal antibody or a monoclonal antibody, but is preferably a monoclonal antibody. A particular example of such a preferred monoclonal antibody is the monoclonal antibody designated as monoclonal antibody 9.3, ATCC No. 10271 described further infra. Preparation of hybridomas producing monoclonal antibody reactive with a specific receptor such as CD28 receptor are known in the art. Methods such as those described by Kohler and Milstein (Nature 256:496 (1975) may be used to produce the hybridomas.

2. Potentiation of the Response by Interleukin-2

Alternatively, the development of cytolytic activity of lymphocytes contacted with anti-CD28 antibody can be further potentiated by treatment with a lymphokine such as IL-2 which is known to stimulate the IL-2 receptor thus activating tyrosine kinase in T cells (Saltzman et al. J. Biol. Chem. 263:6956 (1988); and Morla et al. Mol. Cell. Biol. 8:2214 (1988)). When IL-2 is used, it is preferably present at from about 20 units/ml to about 100 units/ml, most preferably at about 50 units/ml.

3. Contact with CD2 Antibody

The development of cytolytic activity in lymphocytes contacted with anti-CD28 antibody can be further potentiated by contacting the lymphocytes with a second antibody reactive with CD2 receptor. Preferably, the second antibody reactive with CD2 receptor is a monoclonal antibody. A particular example of a preferred monoclonal antibody reactive with the CD2 receptor is the monoclonal antibody designated as 9.6. described further, infra.

## 4. Crosslinking

The effectiveness of the development of the cytolytic activity by anti-CD28 antibody may be increased by aggregating the CD28 receptors in the cell membrane. Such aggregation can be accomplished by crosslinking the anti-CD28 antibody, i.e. reacting the anti-CD28 antibody with a binding partner on the surface of the lymphocytes. The crosslinking may be accomplished by contacting the anti-CD28 antibody with a second antibody reactive with the anti-CD28 antibody. An example of such crosslinking is the use of a rat monoclonal antibody binding to mouse K chains such as monoclonal antibody 187.1, described _infra_, as the second antibody. Alternatively, the CD28 receptors may be aggregated by using a higher molecular weight conjugate of anti-CD28 antibody, for example an anti-CD28/anti-CD28 (9.3 x 9.3) homoconjugate. In addition, the T cells may be contacted with anti-CD28 antibody immobilized to a plastic surface to aggregate the CD28 receptors on the cell surfaces.

## 5. Therapy

Anti-CD28 mAb is useful to activate lymphocytes _in vitro_ and therefore, may be used to regulate cellular immune responses in diseases, infection, cancer, AIDS and autoimmune disorders. Anti-CD28 mAb may be especially useful for the regulation of cellular immune responses in disease states where there is a defect or disregulation of T cells.

The present invention encompasses methods for treating lymphocytes _in vitro_ with anti-CD28 mAb for the regulation of cellular immune responses in disease states. According to one embodiment of the invention, anti-CD28 antibody may be used for the _in vitro_ activation of T cells. This activation can be carried out by contacting T lymphocytes taken from a patient with mAb CD28 _in vitro_ whereby the T cells become activated and can then be reinfused into the autologous donor to kill tumor cells as described by Rosenberg et al., _supra_. Any of the methods of the invention for potentiating the T-cell cytolytic activity using anti-CD28 mAb may be used as the first step in this general method of adoptive therapy. Thus, anti-CD2 antibody or IL-2 may be added with the anti-CD28 antibody to the lymphocytes _in vitro_ to achieve induction of cytolytic activity in the lymphocytes. The CD28 receptor on the lymphocytes is preferably aggregated to enhance the induction of cytolytic activity, for example by crosslinking the anti-CD28 mAb using a second antibody such as 187.1, or with a second anti-CD28 mAb. Alternatively, the anti-CD28 mAb may be immobilized to a plastic surface to aggregate the CD28 receptor for induction of the cytolytic activity. This method of treatment may also involve the _in vitro_ co-incubation or pre-incubation of the T cells with other immunomodulators such as IL-2.

Adoptive therapy has the advantage of avoiding the use of a relatively bulky agent such as a targeting heteroconjugate incorporating an antibody reactive with an antigen associated with a tumor cell. Such heteroconjugates are time consuming and may be difficult to prepare. In adoptive therapy, the lymphocytes are activated _in vitro_ and only the activated lymphocytes are introduced into the subject to kill tumor cells or treat immune disease.

In general, lymphocytes may be activated _in vitro_ for use in adoptive therapy of a patient by treating $10^3$ to $10^8$ lymphocytes with anti-CD28 mAb added at 100 ng/ml of culture fluid to 1 µg/ml. The activated lymphocytes may then be administered to the patient. The most effective mode of administration and dosage regimen for the adoptive therapy will depend on the severity and course of the disease, the patient's health and response to treatment and the judgement of the treating physician. Accordingly, the dosages of the activated lymphocyte should be titrated to the individual patient.

The following examples are presented to illustrate the present invention and to assist one of ordinary skill in making and using the same.

### Example

### Activation of Cytolytic Activity With Anti-CD28 Antibody

### Monoclonal Antibodies

Anti-CD3 mAb G19-4, ATCC No. HP 9536, an IgGl, was obtained as described in Ledbetter et al., _J. Immunol._ 136:3945 (1986). Anti-CD2 mAb 9.6, ATCC No. HB 10267, an IgG2a, was obtained as described in Martin et al., _J. Immunol._ 131:180 (1983). Anti-CD28 mAb 9.3, ATCC No. HB 10271, an IgG2a, was obtained as described in Hansen et al., _Immunogenetics_ 10:247 (1980). Rat mAb 187.1, which binds to mouse K chain, was obtained as described in Yelton et al., _Hybridoma_ 1:5 (1981). Monoclonal antibody L6, ATCC No. HB 8677, an IgG2a, is broadly reactive with carcinomas and was used for targeting to tumor cells to provide heteroconjugates. It was obtained as described in Hellstrom et al,. _Cancer Research_ 46:3917 (1986). _L20, ATCC No. HB 8913_, an IgG1 monoclonal antibody, was not reactive with the H3347 carcinoma cell line used as the target and was used as a control in some experiments. All of these mAbs were purified from ascites fluid before use. Anti-CD16 mAb FC-1, an IgM, was obtained as described in Tetteroo et al., _Leukocyte Typing III_, Ch. M5.3, Michael, Ed. (1987), and was used as ascites fluid at a dilution of 1:

100. Anti-CD2 mAb 9-1, an IgG3, was obtained from Dr. B. Dupont (Memorial Sloan Kettering Cancer Center, N.Y.) as described in Yang et al., J. Immunol. 137:1097 (1986), and was used in purified form. Phorbol-12-myristate-13-acetate (PMA) was obtained from Sigma Chemical Co. (St. Louis, MO).

Preparation of Heteroconjugates

Heteroconjugates were prepared, using maleimidobutyryloxy-succinimide (GMBS; Calbiochem, La Jolla, CA) and imminothiolane HCl (2-IT, Pierce Chemical Co., Rockford, IL) according to a procedure similar to MAb-Phycoerythrin coupling which has been described by Hardy et al., in Methods in Immunology, D. Wein, Ed., p. 31.1 (1986) and was designed to result in little, if any, unconjugated antibody. Briefly, one mAb was treated with 2-IT at 250 $\mu$g/ml mAb and a second mAb was treated with GMBS at 7 $\mu$g/ml mAb. The derivitized mAbs were desalted and mixed together to form a stable thioether bond. Heteroconjugates were analyzed by size exclusion chromatography using a Superose-6 (Pharmacia, Uppsala, Sweden) column (1 X 30 cm). Heteroconjugate sizes ranged from >760 kilodaltons (Kd) to 150 Kd (free antibody). The anti-CD3/L6 mAb heteroconjugate targeting activity was present in all fractions containing conjugates (>150 Kd) and was similar in activity to unfractionated heteroconjugate. Therefore, the anti-CD3/L6 heteroconjugate was used unfractionated in the present experiments.

Preparation of Effector Cells

Human peripheral blood mononuclear cells (PBMCs) were separated from the blood of normal, healthy donors by centrifugation over a Ficoll-Hypaque (Organon Teknika Co., Durham, NC) gradient. After being washed twice in culture medium (CM, RPMI 1640 (Gibco, Grand Island, NY) + 1% L-glutamine + 1% Pen/Strep), cells were incubated at $10^7$/ml with anti-CD16 mAb FC-1 in the form of ascites fluid diluted 1:100 in CM + 15% heat-inactivated pooled normal human serum (PHS) (Pel-Freeze, Brown Deer, WI) for 30 min at 4°C. Rabbit complement (Pel-Freeze) was added at a final dilution of 1:4, and cells were incubated for 45 min at 37°C. For CD3 activation, 75 cm$^2$ flasks were incubated for 60 min at room temperature with 3 ml phosphate buffered saline (PBS) + 10 $\mu$g/ml anti-CD3 mAb G19-4. After 3 washings of the flasks, PBMC samples in CM with 10% PHS were added to the flasks which were then incubated at 37°C. Twenty-four hours before assay, the cells were washed and transferred to fresh flasks with or without addition of IL-2 (Genzyme, Boston, MA). Cells were returned to the incubator until use.

CD2 activation was performed either by a two-epitope method of CD2 stimulation (Yang et al., supra) with two anti-CD2 mAbs, 9.6 and 9-1, or by crosslinking mAb 9.6 on the cell surface with the rat anti-mouse K mAb 187.1. CD28 activation was performed in a similar manner by crosslinking mAb 9.3 on the cell surface with mAb 187.1.

Preparation of Target Cells

H3347, a human colon carcinoma line, and H2981, a human lung carcinoma line, were both developed at Oncogen (Seattle, WA). H3347 is a line that is not lysed by stimulation of T cells with an IgG1 anti-CD3 mAb alone, thus avoiding Fc receptor mediated targeting. They were kept in continuous culture in IMDM$_c$ (Iscove's Modified Dulbecco's Medium (GIBCO, Grand Island, New York) until use. SS, a B-lymphoblastoid cell line (B-LCL) (Oncogen, Seattle, WA) generated by Epstein-Barr Virus (EBV) transformation of normal cells, and K562, an erythroleukemia line, (Oncogen, Seattle, WA) were kept in continuous culture until use. Target cells (3 x $10^6$) were labeled with 250 $\mu$Ci of $^{51}$Cr for 60 min at 37°C and washed 3 times before use.

$^{51}$Cr Release Assay

The $^{51}$Cr release assay measures the cytolytic effect of the effector cells and is described by Jung et al., Proc. Natl. Acad. Sci. USA 83:4479 (1986). Washed effector cells in 50 $\mu$l CM + 10% PHS ($10^7$/ml) were added in triplicate to the wells of a 96-well round bottom plate along with 50 $\mu$l CM + 10% PHS, with or without heteroconjugates, antibodies, or various mixtures of the two. Subsequently, the plates were placed in the incubator at 37°C in a 5% $CO_2$ atmosphere for 4-6 hours. After centrifugation of the plates (400 X g, 10 min), 100 $\mu$l of supernatant were removed from each well. The $^{51}$Cr released from lysed cells was determined using a gamma-ray counter (ICN-Micromedic, Worsham, PA). Percent cell-mediated lysis (CML) was calculated by:

$$\frac{(X\text{-}SPONTANEOUS\ RELEASE)}{(MAXIMAL\ RELEASE\text{-}SPONTANEOUS\ RELEASE)} \times 100$$

where X is the total release of $^{51}$Cr in the presence of the effector cells. Spontaneous release was determined by counting supernatants from labeled target cells incubated only with medium. Maximal release was estimated by exposing labeled target cells to detergent (4% Cetrimide, Sigma Chemical Co., St. Louis, MO) and counting the supernatants. Means of the triplicate determinations are presented herein. Standard errors of the mean did not exceed 10% in any assay. Maximal release averaged 12 times higher in cpm than spontaneous release throughout these experiments.

Depletion of NK Activity by Treatment with Anti-CD16 Antibody Plus Activated Complement

In order to confine the cytolytic activity studied to that of T-cells, CD16-positive cells were removed from PBMCs as a way to eliminate most of the NK cell activity before activating the remaining PBMCs by incubation with immobilized anti-CD3 and IL-2. For this purpose the monoclonal antibody FC-1 (Oncogen, Seattle, WA), an IgM anti-CD16 mAb that fixes complement efficiently, was used.

The H3347 colon carcinoma cell line, which expresses a surface antigen defined by mAb L6, was labelled with $^{51}$Cr and used as targets at an E:T of 50:1 in a five house assay. PBMCs were used as effector cells, either untreated or pretreated with anti-CD16 mAb (FC-1) plus complement (C'). Subsequently, the cells were incubated for 3 days with an anti-CD3 mAb, G19-4, bound to plastic, followed by one day of rest in a fresh flask to which 50 U/ml of IL-2, but no anti-CD3 mAb, had been added. mAb heteroconjugates were used at 3 µg/ml final concentrations and mAb mixtures were used at 1.5 µg/ml for each mAb. Table 1 shows the results in percent CML. The data is representative of six experiments.

TABLE 1

| Lysis of H3347 Carcinoma Cells by an Anti-CD3 L6 Heteroconjugate | | | | | | |
|---|---|---|---|---|---|---|
| | mAb Heteroconjugate or Mixture | | | | | |
| Effector Cell Pre-Treatment | Effectors Alone | Anti-CD3/L6 | Anti-CD3/L20 | Anti-CD28/L6 | Anti-CD3+L6 | L6 Alone |
| None | 21 | 42 | 24 | 18 | 24 | 30 |
| Anti-CD16 + C' | 1 | 14 | 2 | 1 | 2 | 1 |

Resting PBMCs that had been treated with FC-1 plus C' were not at all cytolytic (0% lysis) when tested against the NK-sensitive line K562 at effector cell/target cell (E:T) ratios of from 12.5:1 to 100:1, while a sample of the same PBMCs that had not been treated with FC-1 lysed the K562 target cells (58% lysis). When the CD16-negative cells were activated by anti-CD3 antibody, no lytic activity of effectors alone was generated, whereas effector cells not treated with anti-CD16 and complement retained their lytic activity (Table 1). However, when a conjugate between mAb L6 and the anti-CD3 mAb G19-4 was added to the H3347 carcinoma cells used as targets, these were lysed by activated CD16 negative PBMCs. Addition of unconjugated mAb in equal amounts did not mediate cytotoxicity. Therefore, lysis of H3347 cells was not dependent upon Fc-mediated targeting by the anti-CD3 mAb used in these experiments. Furthermore, the heteroconjugate effect was CD3-independent, because a conjugate between the L6 mAb and the anti-CD28 mAb 9.3 did not mediate target cell killing. Heteroconjugate targeting was also dependent on the specificity of the tumor-cell antibody, L6, since a heteroconjugate of G19-4 mAb and L20, a mAb reactive with lung carcinomas but negative with H3347, did not induce cytotoxicity.

Activation of CD3 Heteroconjugate-Independent Cytolytic Activity by Stimulation with Anti-CD28 Antibody, Anti-CD2 Antibody and Anti-CD3 Antibody

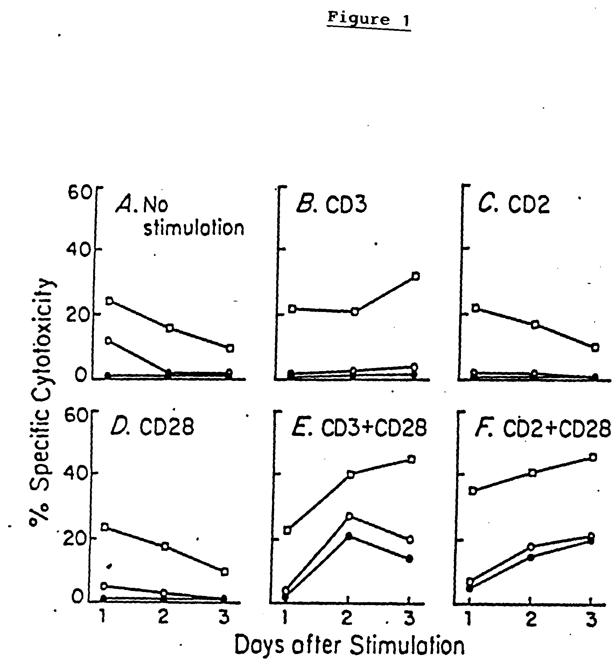

CD16-negative PBMCs were activated over a 3 day period according to six methods before being tested for their ability to lyse $^{51}$Cr labeled H3347 cells (effector to target cell ratio of 50:1) in a 5 hour assay. Activated cells were tested alone with CM + 10% human serum as a control (●—●), with unconjugated mAbs anti-CD3 (G19-4) plus mAb L6 (○—○), or with G19-4/L6 heteroconjugate (☐—☐). The results are shown in Figure 1. When cultured without activation (Figure 1A) the cells progressively lost their ability to lyse targets in the presence of the G19-4/L6 heteroconjugate so that by day 3, less than 10% CML was observed. With the donor used for this experiment, activation by anti-CD2 antibody (9.6 + 9-1, Figure 1C) or anti-CD28 (9.3 crosslinked by 187.1, Figure 1D) pathways also failed to activate a heteroconjugate-dependent lysis, although the PBMCs enlarged and entered the cell cycle. Alternatively, activation with anti-CD3 antibody attached to a solid phase increased the cells' CD3-dependent lytic potential over the 3 day period (Figure 1B), generating over 30% CML by day 3 in the presence of heteroconjugate, while there was no killing in the absence of heteroconjugate.

Addition of anti-CD28 mAb 9.3 to either the anti-CD3 activation (Figure 1E) or the anti-CD2 activation (Figure 1F) not only increased heteroconjugate mediated killing by day 3 (anti-CD3 activated cells increased from 32% to 45%;

anti-CD2 activated cells increased from 10% to 46%), but also exhibited an increased (20%) CD3 heteroconjugate-independent cytolytic activity. These data suggested separate pathways for the CD3 heteroconjugate-dependent and CD3 heteroconjugate-independent lysis. In 7 costimulation experiments with anti-CD3 mAb, anti-CD28 mAb significantly increased CD3 heteroconjugate-independent cytolytic activity (p< .005). Similarly, in 13 costimulation experiments with anti-CD2 mAb, anti-CD28 mAb significantly increased CD3 heteroconjugate-independent cytolytic activity (p< .0005).

Activation of CD3-Independent Cytolytic Activity By Stimulation With IL-2

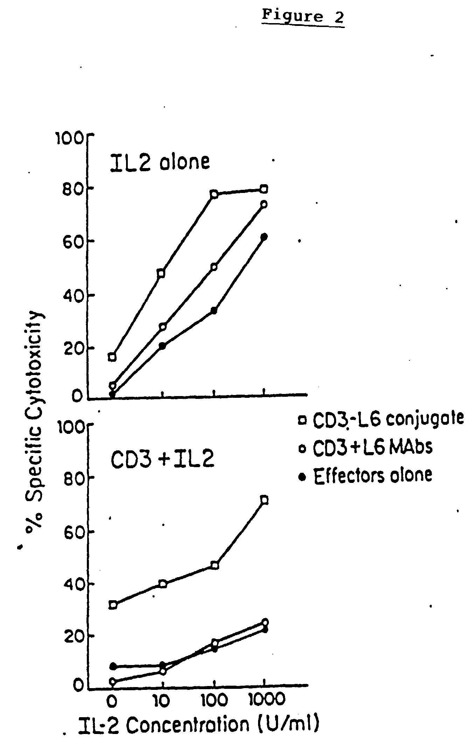

CD16-negative PBMCs were incubated for 4 days alone, or in the presence of increasing concentrations of IL-2, immobilized anti-CD3 mAb, or a combination of the two. Activated cells were tested for their ability to lyse $^{51}$Cr-labeled H3347 tumor targets. Samples were tested in the presence of 3 μg/ml anti-CD3 L6 heteroconjugate (◻━◻ ), 3 μg/ml MAbs anti-CD3 plus L6 ( ◔━◔ ) or alone ( ●━● ) and were observed to generate high levels of CD3 heteroconjugate-independent cytolytic activity (Figure 2). At 1000 U/ml of IL-2, lysis in the presence and absence of the heteroconjugate was 77% and 60% respectively. Lysis was not blocked in this experiment by the addition of 3 μg/ml anti-CD3 antibody. In other experiments concentrations of anti-CD3 mAb up to 100 μg/ml were not able to block this IL-2 generated T-cell killing. Even at low levels of IL-2 (10 U/ml), lysis increased from 2% to 20%. When IL-2 was combined with immobilized anti-CD3 during the activation of resting T-cells, the CD3 heteroconjugate-independent cytolytic activity was reduced. When at 1000 U/ml IL-2, effector cells alone gave only 22% CML, while heteroconjugate-mediated lysis remained high (70%). Additionally, four times as many cells were recovered from the groups activated with anti-CD3 + IL-2 as compared to the groups activated with IL-2 alone. Therefore, CD3-heteroconjugate independent cytolytic activity is induced by IL-2 alone, and is decreased rather than increased by immobilized anti-CD3 mAb.

Comparison of IL-2 and Anti-CD28 Antibody Stimulation of CD3-Independent Cytolytic Activity

CD3 heteroconjugate-independent cytolytic activity was induced by stimulating the CD16-negative cells with increasing concentrations of IL-2 and with anti-CD28 antibody by crosslinking mAb 9.3 on the cell surface with mAb 187.1, as described further below. In addition, CD16-negative cells were stimulated with anti-CD28 and anti-CD2 antibody or PMA.

CD16-negative PBMCs were cultured for 4 days alone or in the presence of 1 μg/ml anti-CD28 mAb (mAb 9.3 crosslinked with mAb 187.1), 1 μg/ml anti-CD2 (mAb 9.6 crosslinked by 187.1) or a combination of the two. Co-stimulation of CD2 and CD28 was performed using crosslinking conditions (as described by Ledbetter et al., E.J. Immunol. 18:1601 (1988)). Briefly, 1 μg/ml of mAb 9.3 was combined with 1 μg/ml of mAb 9.6, then 10 min. later with 8 μg/ml mAb 187.1 with no cell wash. Other samples were activated with PMA (10 ng/ml) or a mixture of anti-CD28 mAb and PMA. IL-2 (50 U/ml) was added in some cultures as indicated. Effector cells were incubated in triplicate with $^{51}$Cr-labeled H3347 colon carcinoma cells at an E:T ratio of 50:1, alone or in the presence of 1.5 μg/ml each of G19-4 mAb (anti-CD3) and L6 mAb. Percent CML in a 5 hour assay is shown in Table 2. The standard errors of the mean did not exceed 10% in any sample.

TABLE 2

| CD28 Stimulation Synergizes with CD2 or PMA Stimulation in Inducing CD3-Independent Cytolytic Activity | | | | |
|---|---|---|---|---|
| Induction of Effector Cells | | %CML | | |
| mAb and/or PMA | IL-2 (50 U/ml) | Effectors alone | CD3/L6 HC | CD3+L6 Mixture |
| None | - | 2 | 21 | 4 |
| | + | 7 | 26 | 12 |
| Anti-CD28 | - | 9 | 25 | 14 |
| (9.3 + 187.1) | + | 21 | 25 | 27 |
| Anti-CD2 | - | 0 | 16 | 2 |
| (9.6 + 187.1) | + | 7 | 17 | 6 |
| Anti-CD2+Anti-CD28 | - | 35 | 46 | 39 |
| (9.3 + 9.6 + 187.1) | + | 36 | 43 | 40 |
| PMA | - | 0 | 9 | 4 |

TABLE 2 (continued)

| CD28 Stimulation Synergizes with CD2 or PMA Stimulation in Inducing CD3-Independent Cytolytic Activity | | | | |
|---|---|---|---|---|
| Induction of Effector Cells | | %CML | | |
| mAb and/or PMA | IL-2 (50 U/ml) | Effectors alone | CD3/L6 HC | CD3+L6 Mixture |
| | + | 9 | 22 | 21 |
| Anti-CD28(9.3)+PMA | - | 25 | 41 | 33 |

As shown in Table 2, cells cultured with anti-CD28 antibody slightly increased the CD3 heteroconjugate-independent cytolytic activity (9% versus 2%) slightly above background levels, as did activation with low levels of IL-2 (7% versus 2%). However, co-stimulation with CD28 and IL-2 increased the CD3 heteroconjugate-independent cytolytic activity to 21%. In each of these cases, specific heteroconjugate-mediated lysis was essentially unchanged. Crosslinking of an anti-CD2 mAb, alone or in combination with IL-2, induced only minimal cytolytic activity.

Co-stimulation of CD2 and CD28 generated both CD3 heteroconjugate-dependent (46% CML) and heteroconjugate independent (35% CML) activity, which was not further increased by the addition of IL-2 during the 4 day activation. Addition of PMA to the anti-CD28 MAb increased the generation of both the CD3 heteroconjugate-independent and dependent cytolytic activity, while incubation of cells with PMA alone during activation was ineffective. Addition of IL-2 to PMA during the activation period slightly increased the cells' cytolytic ability.

Table 2 thus shows that anti-CD28 antibody can stimulate a significant amount of cytolytic activity independent of the presence of a CD3 heteroconjugate, that is not inhibited by unconjugated anti-CD3 mAb. This stimulation is further increased by the addition of PMA, IL-2, or anti-CD2 antibody. Co-stimulation of cells with anti-CD28 mAb together with IL-2 or in combination with immobilized anti-CD3 mAb was able to elicit or augment a CD3 heteroconjugate-independent cytolytic response as shown in this example. Incubation of cells with IL-2 alone generated high levels of cytolytic activity which was CD3 heteroconjugate-independent and was not inhibited by anti-CD3 mAb.

Although co-stimulation of T-cells with anti-CD28 mAb has been reported to produce high levels of IL-2 (Martin, et al., J. Immunol. 136:3282 (1986)), the results presented herein demonstrate that incubation with anti-CD28 mAb 9.3 plus low levels of IL-2 (10 units/ml) gave a higher cytolytic response than incubation with IL-2 or anti-CD28 mAb individually, suggesting that the generation of cytolytic activity by exposure to an anti-CD28 mAb may not be mediated by IL-2 production alone. This is also supported by IL-2 vs. CD28 signal transduction differences, and differences in the interaction with anti-CD3 mAb.

The anti-CD28 antibody may thus be used to induce cytolytic activity in lymphocytes in the presence of a co-stimulatory agent such as anti-CD2 mAb, or IL-2. In addition, in contrast to heteroconjugate-mediated killing which is dependent on stimulation of the CD3 receptor, the cell-killing phase of the cytolytic activity of lymphocytes stimulated with anti-CD28 mAb occurs in the absence of heteroconjugates containing anti-CD3 mAb and a tumor targeting antibody and does not require stimulation of the CD3 receptor.

**Claims**

1. A method of potentiating the development of CD3 independent cytolytic activity of CD28+ lymphocytes comprising the step of contacting the lymphocytes *in vitro* with an antibody reactive with CD28 receptor to produce cytolytic lymphocytes capable of attacking and lysing tumor cells.

2. The method of claim 1 wherein said lymphocytes are T lymphocytes.

3. The method of Claim 1 or 2, wherein the lymphocytes are CD16 depleted lymphocytes.

4. The method of any preceding further comprising the step of contacting said lymphocytes with antibody reactive with CD2 receptor, and/or with IL-2.

5. The method of Claim 4 further comprising the step of crosslinking said antibody reactive with CD2 receptor.

6. The method of any preceding claim, wherein the antibody reactive with the CD28 or CD2 receptor is a monoclonal antibody.

7. The method of claim 6, wherein the monoclonal antibody reactive with the CD28 receptor is the monoclonal antibody produced by the hybridoma deposited with the American Type Culture Collection and designated HB 10271 and the monoclonal antibody reactive with the CD2 receptor is the monoclonal antibody produced by the hybridoma deposited with the ATCC and designated HB 10267.

8. The method of any preceding claim, wherein the antibody reactive with the CD28 receptor is cross-linked to aggregate the CD28 receptor.

9. The method of claim 8, wherein the cross-linking of the antibody reactive with the CD28 receptor is carried out by a second antibody reactive with the antibody reactive with the CD28 receptor.

10. The method of claim 9, wherein the second antibody is a rat monoclonal antibody reactive with mouse kappa chain.

11. The method of claim 8, wherein the cross-linking is carried out by contacting the lymphocytes with a high molecular weight conjugate.

12. The method of claim 11, wherein the high molecular weight conjugate comprises monoclonal antibody reactive with CD28 receptor.

13. Use *in vitro* of an antibody reactive with CD28 receptor for potentiating the development of CD3 independent cytolytic activity of CD28$^+$ lymphocytes to produce cytolytic lymphocytes capable of attacking and lysing tumor cells.

**Patentansprüche**

1. Verfahren zur Potenzierung der Entwicklung der CD3-unabhängigen cytolytischen Aktivität von CD28$^+$-Lymphocyten, wobei man die Lymphocyten *in vitro* mit einem gegenüber dem CD28-Rezeptor reaktiven Antikörper in Kontakt bringt, um cytolytische Lymphocyten zu produzieren, die in der Lage sind, Tumorzellen anzugreifen und zu lysieren.

2. Verfahren nach Anspruch 1, wobei die Lymphocyten T-Lymphocyten sind.

3. Verfahren nach Anspruch 1 oder 2, wobei die Lymphocyten CD16-depletierte Lymphocyten sind.

4. Verfahren nach einem der vorhergehenden Ansprüche, wobei man die Lymphocyten mit einem gegenüber dem CD2-Rezeptor reaktiven Antikörper und/oder mit IL-2 in Kontakt bringt.

5. Verfahren nach Anspruch 4, welches als weiteren Schritt die Vernetzung des gegenüber dem CD2-Rezeptor reaktiven Antikörpers umfaßt.

6. Verfahren nach einem der vorhergehenden Ansprüche, wobei der gegenüber dem CD28- oder CD2-Rezeptor reaktive Antikörper ein monoklonaler Antikörper ist.

7. Verfahren nach Anspruch 6, wobei der gegenüber dem CD28-Rezeptor reaktive monoklonale Antikörper derjenige monoklonale Antikörper ist, der von dem Hybridom produziert wird, das bei der American Type Culture Collection hinterlegt ist und als HB 10271 bezeichnet wird, und der gegenüber dem CD2-Rezeptor reaktive monoklonale Antikörper derjenige monoklonale Antikörper ist, der von dem Hybridom produziert wird, das bei der ATCC hinterlegt ist und als HB 10267 bezeichnet wird.

8. Verfahren nach einem der vorhergehenden Ansprüche, wobei der gegenüber dem CD28-Rezeptor reaktive Antikörper vernetzt wird, um den CD28-Rezeptor zu aggregieren.

9. Verfahren nach Anspruch 8, wobei die Vernetzung des gegenüber dem CD28-Rezeptor reaktiven Antikörpers mit einem zweiten Antikörper durchgeführt wird, welcher gegenüber dem Antikörper reaktiv ist, der mit dem CD28-Rezeptor reagiert.

10. Verfahren nach Anspruch 9, wobei der zweite Antikörper ein monoklonaler Ratte-Antikörper ist, der mit der Maus-Kappakette reagiert.

**11.** Verfahren nach Anspruch 8, wobei die Vernetzung dadurch erfolgt, daß man die Lymphocyten mit einem hochmolekularen Konjugat in Kontakt bringt.

**12.** Verfahren nach Anspruch 11, wobei das hochmolekulare Konjugat monoklonale Antikörper umfaßt, die mit dem CD28-Rezeptor reagieren.

**13.** *In vitro*-Verwendung eines gegenüber dem CD28-Rezeptor reaktiven Antikörpers zur Potenzierung der Entwicklung der CD3-unabhängigen cytolytischen Aktivität von CD28$^+$-Lymphocyten, um cytolytische Lymphocyten zu produzieren, die in der Lage sind, Tumorzellen anzugreifen und zu lysieren.

**Revendications**

**1.** Méthode de potentialisation du développement de l'activité cytolytique indépendante de CD3 des lymphocytes CD28$^+$ comprenant l'étape de mise en contact des lymphocytes *in vitro* avec un anticorps réactif avec le récepteur de CD28 pour produire des lymphocytes cytolytiques capables d'attaquer et de lyser des cellules tumorales.

**2.** Méthode selon la revendication 1 où lesdits lymphocytes sont des lymphocytes T.

**3.** Méthode selon la revendication 1 ou 2, où les lymphocytes sont des lymphocytes CD16 épuisés.

**4.** Méthode selon l'une quelconque des revendications précédentes comprenant de plus l'étape de mise en contact desdits lymphocytes avec un anticorps réactif avec le récepteur de CD2, et/ou avec IL-2.

**5.** Méthode selon la revendication 4 comprenant de plus l'étape de réticulation dudit anticorps réactif avec le récepteur de CD2.

**6.** Méthode selon l'une quelconque des revendications précédentes, dans laquelle l'anticorps réactif avec le récepteur de CD28 ou CD2 est un anticorps monoclonal.

**7.** Méthode selon la revendication 6, où l'anticorps monoclonal réactif avec le récepteur de CD28 est l'anticorps monoclonal produit par l'hybridome déposé auprès de l'American Type Culture Collection et désigné HB 10271 et l'anticorps monoclonal réactif avec le récepteur de CD2 est l'anticorps monoclonal produit par l'hybridome déposé auprès de l'ATCC et désigné HB 10267.

**8.** Méthode selon l'une quelconque des revendications précédentes, où l'anticorps réactif de récepteur de CD28 est réticulé pour aggréger le récepteur de CD28.

**9.** Méthode selon la revendication 8, où la réticulation de l'anticorps réactif avec le récepteur de CD28 est mise en oeuvre par un second anticorps réactif avec l'anticorps réactif avec le récepteur de CD28.

**10.** Méthode selon la revendication 9, où le second anticorps est un anticorps monoclonal de rat réactif avec la chaîne kappa de souris.

**11.** Méthode selon la revendication 8, dans laquelle la réticulation est mise en oeuvre par mise en contact des lymphocytes avec un conjugué à haut poids moléculaire.

**12.** Méthode selon la revendication 11, où le conjugué à haut poids moléculaire comprend un anticorps monoclonal réactif avec le récepteur de CD28.

**13.** Utilisation *in vitro* d'un anticorps réactif avec le récepteur de CD28 pour potentialiser le développement de l'activité cytolytique indépendante de CD3 des lymphocytes CD28$^+$ pour produire des lymphocytes cytolytiques capables d'attaquer et de lyser des cellules tumorales.

Figure 1

Figure 2